# EUROPEAN PATENT SPECIFICATION

(11) **EP 3 266 390 B1**
(45) Date of publication and mention of the grant of the patent: **05.08.2020**
(21) Application number: 16759006.6
(22) Date of filing: 03.03.2016
(51) Int. Cl.: A61B 17/12, A61M 37/00, A61L 31/02, A61L 31/16, C23C 16/56, A61L 31/04, A61B 17/00, A61L 31/14

(54) **VASCULAR EMBOLIZATION TOOL AND PRODUCTION METHOD THEREFOR**
GEFÄSSEMBOLISATIONSINSTRUMENT UND HERSTELLUNGSVERFAHREN DAFÜR
INSTRUMENT D'EMBOLISATION DE VAISSEAU SANGUIN, ET PROCÉDÉ DE FABRICATION DE CELUI-CI

(30) Priority: 03.03.2015 JP 2015041625
(43) Date of publication of application: 10.01.2018
(73) Proprietor: Kaneka Medix Corporation, Osaka-shi, Osaka 530-8288 (JP)
(72) Inventor: OZASA, Hitoshi, Settsu-shi Osaka 566-0072 (JP); OGAWA, Atsushi, Osaka, 566-0072 (JP); YAMANAKA, Yasushi, Settsu-shi Osaka 566-0072 (JP); IWATA, Hiroo, Osaka 618-0024 (JP); KODAMA, Tomonobu, Tokyo 135-0061 (JP)
(74) Representative: TBK
(86) International application number: PCT/JP2016/056618
(87) International publication number: WO 2016/140314

(56) References cited:
- EP-A1- 2 316 355
- EP-A2- 1 691 721
- WO-A1-2004/073528
- JP-A- H1 176 249
- JP-A- 2003 501 131
- JP-A- 2007 523 055
- JP-A- 2013 537 046
- JP-A- 2015 501 702
- US-A1- 2007 142 859
- US-A1- 2011 118 772
- US-A1- 2013 072 959
- US-A1- 2013 131 711

## Description

### TECHNICAL FIELD

The present invention relates to a vascular embolization device to be placed at a predetermined site in a blood vessel and to embolize the blood vessel.

### BACKGROUND ART

Less invasive therapies widely used to treat, for example, aneurysm include vascular embolization in which an embolization device is placed in the dome (Patent Literatures 1 and 2). In the vascular embolization, the embolization device placed in the aneurysm serves as a physical barrier against blood flow and causes the formation of a thrombus around the embolization device, so that the risk of aneurysm rupture will decrease. A known embolization device to be placed at a predetermined site in a blood vessel, such as aneurysm, includes a metal coil (hereinafter such a device will also be called an "embolization coil") (Patent Literatures 3 to 5). Such an embolization coil is introduced into the aneurysm through an appropriate catheter by pushing means (an introducer), which is detachably connected to an end of the embolization coil.

In some target sites (cases) where the embolization coil is to be placed, however, a biochemical active material needs to be administered to the target sites.

For example, if no biochemical active material is administered, biological tissues may insufficiently form around the embolization coil placed in the aneurysm, so that the blood may enter the aneurysm to cause recanalization, which may enlarge the dome again. Therefore, it is preferable that an organization promoter be administered into the aneurysm to promote the formation of biological tissues around the embolization coil placed in the aneurysm.

Patent Literature 6 describes an embolization device capable of administering a biochemical active material or a drug, which includes a metal coil and a resin wire that is provided over the inside of the metal coil and contains the biochemical active material or the drug. Patent Literature and 8 disclose vascular embolization devices as specified in the preamble of claim 1.

### CITATIONS LIST

### PATENT LITERATURES

Patent Literature 1: US 4 884 579 A
Patent Literature 2: 4 739 768 A
Patent Literature 3: JP H05-500322 A
Patent Literature 4: JP H08-501015 A
Patent Literature 5: JP H07-502674 A
Patent Literature 6: JP H11-76249 A
Patent Literature 7: EP 2 316 355 A1
Patent Literature 8: US 2013/131711

### SUMMARY OF INVENTION

### TECHNICAL PROBLEMS

However, the addition of a biochemical active material to a wire made of a single resin material can cause the resin wire to have insufficient flexibility depending on the composition of the resin, which means that there is room for improvement in ease of handling vascular embolization devices to be inserted into blood vessels.

It is an object of the present invention to provide a vascular embolization device having the function of administering a biochemical active material and having good flexibility.

### SOLUTIONS TO PROBLEMS

As a measure to solve the above problems, the inventors have considered a method of inserting a polyvinyl alcohol-based resin wire containing a biochemical active material into the inside of a coil, as described in Patent Literature 6, to allow a vascular embolization device to carry the biochemical active material. However, there has been a problem in that the addition of a biochemical active material to a polyvinyl alcohol-based resin reduces the strength of the resin itself depending on the composition of the resin, and makes it difficult to draw the resin into a linearly elongated shape. This results in difficulty in forming the resin into a wire and difficulty in inserting the resin into a coil. There has also been a problem in that if the wire is made thick in order to compensate for that, the coil will be too hard to be practically used.

As solutions to the above problems, the inventors have come up with first forming only a resin wire and then coating the resin wire with a biochemical active material-containing resin, in which resins with solubilities different from each other in the same organic solvent are used for forming the resin wire and for the coating, respectively, so that a resin wire containing a biochemical active material, which has a constant strength and is insertable into a coil can be formed.

As a result of intensive studies to solve the above problems, the inventors have accomplished the present invention.

Specifically, the present invention provides a vascular embolization device as defined by claim 1 and a vascular embolization device producing method as defined by claim 22.

### ADVANTAGEOUS EFFECTS OF INVENTION

The present invention makes it possible to provide a vascular embolization device having the function of administering a desired biochemical active material to target sites and also having good flexibility so that it can provide a good embolization effect at various target sites in blood vessels.

### BRIEF DESCRIPTION OF DRAWINGS

FIG. 1 is a cross-sectional view showing an example of a specific structure of a vascular embolization device according to the present invention.
FIG. 2 is a cross-sectional view showing another example of a specific structure of the vascular embolization device according to the present invention.
FIG. 3 is a cross-sectional view showing another example of a specific structure of the vascular embolization device according to the present invention.
FIG. 4 is an explanatory view showing the cross-section of a resin wire 20 constituting a vascular embolization device 1 according to the present invention.
FIG. 5 is an explanatory view showing an example of a secondary shape formed by winding, into a coil, the vascular embolization device 1 according to the present invention.
FIG. 6 is an explanatory view showing the connection of pushing means 30 to the vascular embolization device 1 according to the present invention.
FIG. 7 is an explanatory view showing how to use the vascular embolization device 1 according to the present invention in the human body.
FIG. 8 is an explanatory view showing a method for measuring the coil flexibility of the vascular embolization devices prepared in Example 1 and Comparative Example 1.
FIG. 9 is a graph showing the results of measurement of the coil flexibility of the vascular embolization devices prepared in Example 1 and Comparative Example 1.
FIG. 10 is a cross-sectional view showing another example of a specific structure of the vascular embolization device according to the present invention.
FIG. 11 is a cross-sectional view showing another example of a specific structure of the vascular embolization device according to the present invention.

### DESCRIPTION OF EMBODIMENTS

Hereinafter, a vascular embolization device according to an embodiment of the present invention will be described, with reference to the drawings. It will be understood that such an embodiment is not intended to limit the present invention.

### (Vascular embolization device)

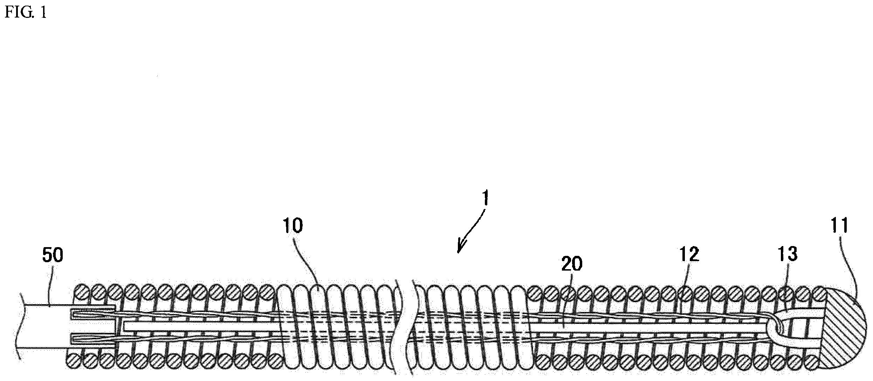

FIG. 1 is a cross-sectional view showing an example of a specific structure of a vascular embolization device 1 according to the present invention.

The vascular embolization device 1 includes a coil 10 and a resin wire 20 inserted in the inside of the coil 10.

### (Coil)

The coil 10 constituting the vascular embolization device 1 is preferably made of an X-ray-impermeable metal wire, such as a wire of any of platinum, gold, tungsten, iridium, palladium, rhodium, indium, iron, nickel, cobalt, chromium, manganese, molybdenum, aluminum, titanium, niobium, silicon, metal phosphide, sulfide mineral, zirconium, copper, stainless steel, and alloys thereof.

The wire used to form the coil 10 preferably has a diameter (wire diameter) of 0.02 mm to 0.12 mm, more preferably 0.03 mm to 0.10 mm, in view of its insertion or placement in blood vessels. The coil 10 preferably has a diameter of 0.1 mm to 1.0 mm, more preferably 0.2 mm to 0.5 mm, in view of its insertion or placement in blood vessels.

In the vascular embolization device 1, the coil 10 preferably has a length of 1 mm to 1,000 mm, more preferably 1 mm to 500 mm, even more preferably 10 mm to 500 mm.

### (Resin wire)

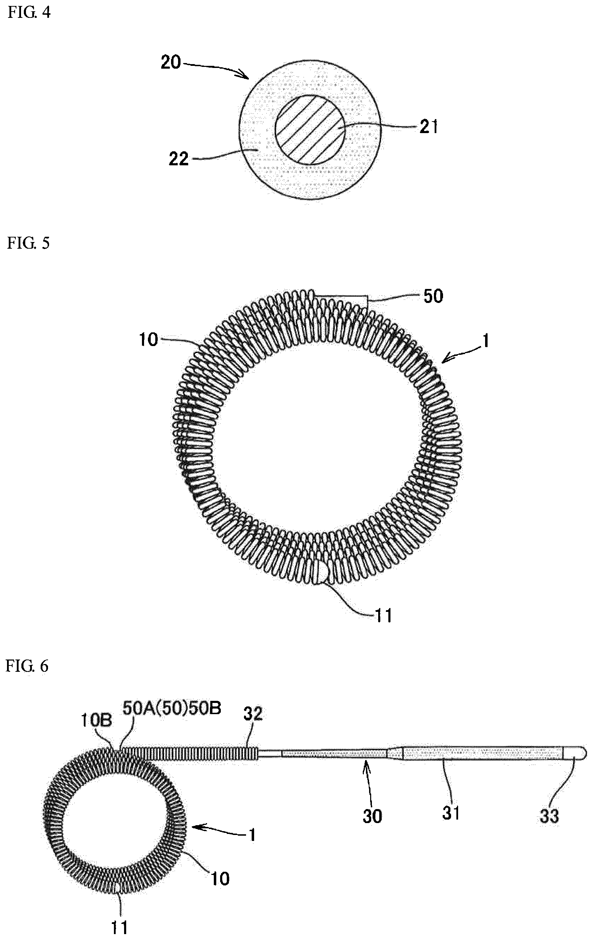

As shown in the cross-sectional view of FIG. 4, the resin wire 20 is a multilayer strand including a core 21 and at least one outer layer 22 with which at least part of the surface of the core 21 is covered.

In FIG. 4, a single outer layer 22 is provided. Alternatively, two or more outer layers 22 may be provided to form a multilayer structure.

Although not shown, the resin wire 20 may include the core 21 which has two resin wires or three or more resin wires.

The thickness of the resin wire 20 (including the thicknesses of the core 21 and the outer layer 22) may be any thickness that makes the resin wire 20 insertable into the inside of the coil 10. For example, the thickness of the resin wire 20 is preferably from 0.01 mm to 0.20 mm, more preferably from 0.06 mm to 0.15 mm, even more preferably from 0.08 mm to 0.12 mm.

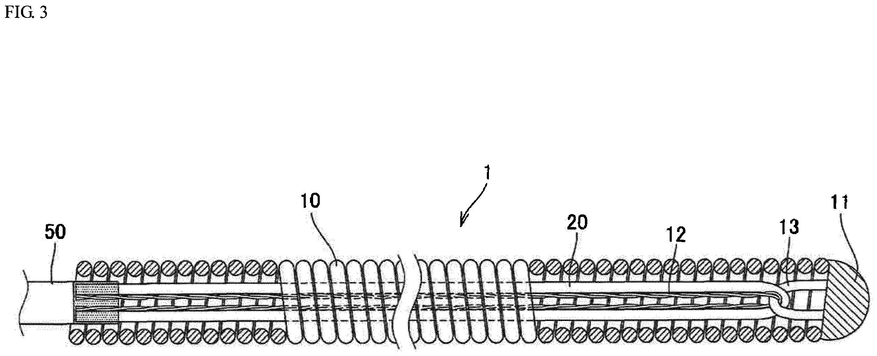

In this regard, two or more resin wires 20 may be inserted in the inside of the coil 10. For example, as shown in FIG. 3, one resin wire 20 may be inserted into the inside of the coil 10 and then folded back at the ring part 13 of a tip 11 at the front end, so that the resin wire 20 may be double-folded and inserted in the inside of the coil 10.

The resin wire 20 may be linearly-shaped as shown in FIG. 1 or may be partially or entirely corrugated or spirally shaped. The corrugated or spirally-shaped resin wire 20 can have a greater entire length and thus contain a larger amount of a biochemical active material than the corresponding linearly-shaped resin wire has.

The corrugated shape may be, for example, a substantially sine wave shape or a substantially square wave shape.

The spiral shape may be any helical shape with a size enough to fit in the inside of the coil 10.

The present invention has the feature that a core 21 and at least one outer layer 22 in the resin wire 20 include resin compositions with solubilities different from each other in the same organic solvent.

The core 21 and the outer layer 22 are formed by resin compositions with solubilities different from each other in the same organic solvent, to thereby stably form the outer layer 22 without dissolution and breakage of the resin wire for forming the core 21, even when the surface of the core 21 is coated with a resin composition for forming the outer layer 22.

In the present invention, any type of organic solvent capable of dissolving the resin for forming the resin wire 20 may be used in the evaluation of the solubility, and the type of the organic solvent is not particularly limited. The term "solubility" refers to the solubility attained when the resin wire 20 or the resin for forming the resin wire 20 is immersed in the organic solvent. Specifically, the solubility can be determined by the method described below in the section entitled "EXAMPLES."

In order to form the outer layer 22 by coating without dissolution and breakage of the core 21-forming resin wire, it is particularly preferable that the resin composition contained in the outer layer 22 should have a solubility higher than that of the resin composition contained in the core 21.

In addition, the resin used to form either the core 21 or the outer layer 22 of the resin wire 20 is preferably selected from resins with good flexibility (such a degree of flexibility as to be easily deformable by the shape retaining force of the coil 10) and no adverse effect on the living body.

The resin composition for forming either the core 21 or the outer layer 22 includes an ethylene-vinyl acetate copolymer, a styrene-isobutylene-styrene block copolymer, a styrene-ethylene-propylene-styrene block copolymer, a styrene-ethylene-butadiene-styrene block copolymer, and a styrene-ethylene-ethylene-propylene-styrene block copolymer.

Among them, an ethylene-vinyl acetate copolymer can surely hold a necessary and sufficient amount of a biochemical active material, have sufficient flexibility, and properly release the biochemical active material upon contact with blood. From these points of view, the resin composition for forming either the core 21 or the outer layer 22 preferably includes an ethylene-vinyl acetate copolymer.

Particularly in order to prevent delamination of the coating layer, a resin composition including an ethylene-vinyl acetate copolymer is preferably used to form each of the core 21 and the outer layer 22.

Resin compositions each including an ethylene-vinyl acetate copolymer may be used to form the core 21 and the outer layer 22, respectively. In this case, the ethylene-vinyl acetate copolymers used to form the core 21 and the outer layer 22, respectively, should preferably have different compositions so that the surface of the resin wire for forming the core 21 can be successfully coated with the outer layer 22 without dissolution and breakage of the resin wire for forming the core 21.

When such ethylene-vinyl acetate copolymers with different compositions are used, for example, the copolymer selected for the core 21 should preferably have a vinyl acetate unit content of 10% to 30%, and the copolymer selected for the outer layer 22 should preferably have a vinyl acetate unit content of 30% to 50%.

In the present invention, the vinyl acetate unit content of ethylene-vinyl acetate copolymers can be measured by saponification method (provided in Japanese Industrial Standards (JIS K 7192)), infrared spectroscopy (IR spectroscopy), nuclear magnetic resonance spectroscopy (NMR spectroscopy), or thermogravimetry.

The resin wire 20 constituting the vascular embolization device 1 contains at least one biochemical active material.

In the present invention, the " resin wire containing a biochemical active material" is intended to include (1) a resin wire containing a biochemical active material uniformly dissolved or dispersed in the resin, (2) a resin wire containing a biochemical active material localized at and near the surface of the wire-shaped resin material (e.g., a resin wire containing a biochemical active material applied to the surface of the resin), and (3) a resin wire containing a biochemical active material localized at the center of the wire-shaped resin material (e.g., a resin wire containing a biochemical active material (inner part) covered with a resin film (outer part)).

Specifically, the biochemical active material may be contained in the core 21 or at least one outer layer 22 depending on the purpose.

For example, the biochemical active material should preferably be contained in at least one outer layer 22, particularly, in the outermost layer, so that the release of the biochemical active material can be completed in a short period of time including the initial burst release period.

In addition, the biochemical active material should preferably be contained in the core 21 or in at least one core 21 and at least one outer layer 22, so that the biochemical active material can be continuously released over a long period of time.

When two or more outer layers 22 are provided to form a multilayer structure, the position of a layer containing the biochemical active material may be controlled so that the duration of release of the biochemical active material can be controlled. For example, a resin composition layer including only a resin or having a lower content of the biochemical active material may be formed on the outer side of the biochemical active material-containing layer, so that the biochemical active material can be continuously released at a lower rate over a longer period of time as compared to the case where the outermost layer contains the biochemical active material.

The biochemical active material contained in the core 21 or the outer layer 22 may be uniformly distributed over the core 21 or the outer layer 22 or may be localized at a predetermined site such as the center or periphery of the core 21 or the outer layer 22.

The biochemical active material may be an organization promoter, a blood coagulation accelerator, an anticancer drug, or any other drug according to the desired purpose.

For example, an organization promoter may be added to the resin for forming the resin wire 20, so that the resulting vascular embolization device 1 can promote the formation of biological tissues when placed in the aneurysm to be embolized.

In the present invention, such an organization promoter is preferably any of statins such as simvastatin, pravastatin, atorvastatin, pitavastatin, fluvastatin, lovastatin, and rosuvastatin. Particularly for promotion of endothelium formation at the entrance (neck) of aneurysm, the organization promoter is preferably simvastatin, pravastatin, atorvastatin, pitavastatin, or any combination thereof.

A blood coagulation accelerator may also be added to the resin for forming the resin wire 20, so that vascular closure can be accelerated in the blood vessel where the resulting vascular embolization device 1 is used for embolization. Examples of such a blood coagulation accelerator include coagulation accelerators such as phytonadione, protamine sulfate, hemocoagulase, and menatetrenone, external hemostatics such as sodium alginate, gelatin, and oxidized cellulose, blood coagulation factor preparations such as eptacog alfa, octocog alfa, turoctocog alfa, desmopressin acetate hydrate, thrombin, and rurioctocog alfa, sclerosants for esophageal varices, such as oleic acid monoethanolamine and polidocanol, antiplasmin agents such as tranexamic acid, and capillary stabilizers such as ascorbic acid, carbazochrome, phytonadione, carbazochrome sodium sulfonate hydrate, and adrenochrome monoaminoguanidine mesilate.

An anticancer drug may also be added to the resin for forming the resin wire 20, which makes it possible to accelerate degeneration and elimination of cancer tissues around the blood vessel where the resulting vascular embolization device 1 is used for embolization. Examples of such an anticancer drug include alkaloids such as paclitaxel, cytochalasin, docetaxel, vincristine, vinblastine, vinorelbine, etoposide, teniposide, misplatin, vindesine, and irinotecan; antibiotics such as mitomycin, adriamycin, doxorubicin, actinomycin, daunorubicin, idarubicin, mitoxantrone, bleomycin, plicamycin, aclarubicin, pirarubicin, epirubicin, peplomycin, neocarzinostatin, and zinostatin stimalamer; alkylating agents such as nitrogen mustard, mechlorethamine, cyclophosphamide, melphalan, chlorambucil, ethyleneimine, thiotepa, methyl melanin, busulfan, carmustine, streptozocin, dacarbazine, procarbazine, carboquone, nimustine, ranimustine, mitobronitol, and temozolomide; antimetabolites such as methotrexate, fluorouracil, floxuridine, cytarabine, mercaptopurine, thioguanine, mentostatin, chlorodeoxyadenosine, hydroxycarbamide, Starasid ocfosphate, enocitabine, fludarabine, gemcitabine, doxifluridine, Tegafur, Tegafur uracil, Levofolinate, carmofur, methotrexate, TS One (registered trademark), and capecitabine; platinum-based agents such as cisplatin, carboplatin, and nedaplatin; hormone drugs such as leuprorelin, goserelin, medroxyprogesterone, tamoxifen, toremifene citrate, fadrozole, estramustine sodium phosphate ester, flutamide, and bicalutamide; retinoids such as tretinoin; and other drugs such as imatinib, dasatinib, nilotinib, gefitinib, folinate, mozavaptane, sunitinib, sorafenib, axitinib, lapatinib, azathioprine, cyclosporine, tacrolimus, sirolimus, zotarolimus, everolimus, Biolimus A9, mycophenolate mofetil, mizoribine, calcipotriol, gusperimus, muromonab-CD3, thalidomide, lenalidomide, bicalutamide, aceglatone, octreotide, pentostatin, sobuzoxane, porfimer sodium, tranilast, sodium aurothiomalate, penicillamine, lobenzarit, bucillamine, losartan potassium, candesartan cilexetil, valsartan, lisinopril, captopril, cilazapril, enalapril, temocapril hydrochloride, quinapril hydrochloride, trandolapril, delapril hydrochloride, perindopril erbumine, nifedipine, nilvadipine, efonidipine hydrochloride, felodipine, and colchicine.

The content of the biochemical active material in the resin wire 20 is preferably from 1 part by weight to 99 parts by weight, more preferably from 1 part by weight to 60 parts by weight, even more preferably from 1 part by weight to 50 parts by weight, based on 100 parts by weight of the resin.

### (Stretch resistant wire)

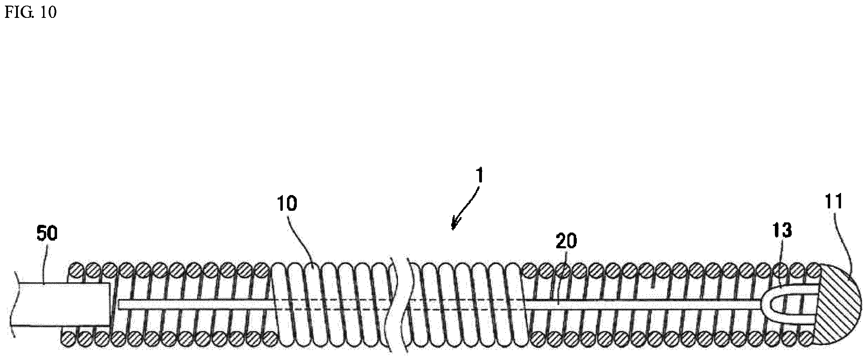

The vascular embolization device 1 of the present invention, which has the resin wire 20 inserted in the inside of the coil 10 as shown in FIG. 10, may further include, as shown in FIG. 1, a stretch resistant wire 12 provided in the inside of the coil 10.

In this case, the stretch resistant wire 12 is preferably fixed at the front and rear ends of the coil 10 so that it can prevent the coil 10 from stretching when the coil 10 is placed at the target site in the blood vessel.

The stretch resistant wire 12 may have any thickness that allows it to be inserted into the inside of the coil 10, and thus the thickness is not particularly limited. In view of the balance between properties such as stretch resistant properties and flexibility, the stretch resistant wire 12 preferably has a thickness of 0.01 mm to 0.10 mm, more preferably 0.01 mm to 0.06 mm.

In order to prevent the stretching of the coil 10, the stretch resistant wire 12 preferably has a break strength of 0.05 N or more per wire, more preferably 0.10 N or more per wire.

The break strength can be measured using a tensile tester.

The stretch resistant wire 12, which should be linear as shown in FIG. 1, may be partially or entirely corrugated or spirally-shaped.

The stretch resistant wire 12 corrugated or spirally-shaped partially or entirely can resist straightening (a phenomenon in which when the coil 10 is folded into a compact form and placed in the living body, the stretch resistant wire 12 becomes not long enough so that an end of the coil 10 becomes stiff), so that the coil 10 can be placed in a wider variety of forms depending on the situation in the body. In addition, the corrugated or spirally-shaped stretch resistant wire 12 can have an entire length greater than that of the straight type, so that a larger amount of the biochemical active material can be held inside the coil 10 when the biochemical active material is carried by the stretch resistant wire 12 as described later.

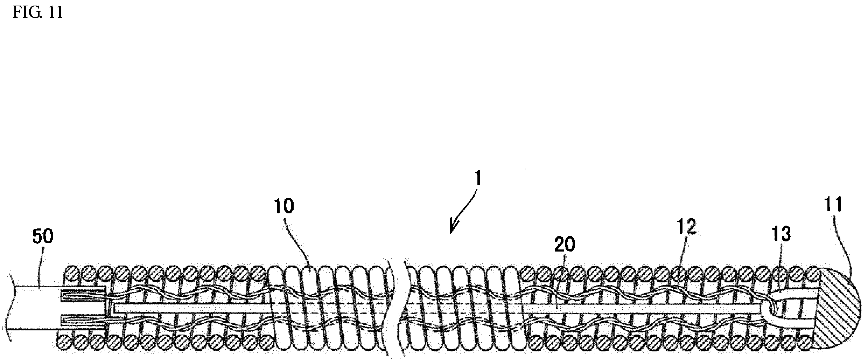

The corrugated shape may be, for example, a substantially sine wave shape or a substantially square wave shape. For example, as shown in FIG. 11, the stretch resistant wire 12 may be substantially sine wave-shaped over the entire length.

The spiral shape may be any helical shape with a size enough to fit in the inside of the coil 10.

The stretch resistant wire 12 prevents excessive stretching of the coil 10 while allowing the coil 10 to deform flexibly. From this point of view, the natural length of the stretch resistant wire 12 is preferably adjusted to be at least 5% longer, more preferably at least 10% longer, even more preferably at least 20% longer than the natural length of the coil 10.

In this regard, the natural length of the stretch resistant wire 12 is a longitudinal length of the stretch resistant wire 12. For example, as shown in FIG. 1, it refers to the length of the stretch resistant wire 12 between the ring part 13 and the connection part 50 when the stretch resistant wire 12 is a stranded wire composed of two strands.

The stretch resistant wire 12 may be made of a metal or a resin composition. Examples of the material for the stretch resistant wire 12 include: metals such as platinum, gold, tungsten, tantalum, iridium, palladium, rhodium, indium, iron, nickel, cobalt, chromium, manganese, molybdenum, aluminum, titanium, niobium, silicon, metal phosphide, sulfide mineral, zirconium, copper, stainless steel, and alloys thereof; polymers such as polyethylene, polypropylene, polyethylene terephthalate, polyamide, polyester, polylactic acid, polyglycolic acid, poly(lactic acid-glycolic acid) copolymers, polyhydroxybutyric acid, polyhydroxybutyrate valeric acid, 3-hydroxybutyric acid-3-hydroxyhexanoic acid copolymer polyester; and polymers derived from biodegradable polymers, such as cellulose, polydioxanone, proteins, and vinyl polymers.

Particularly, in view of biocompatibility, gold, platinum, iridium, tungsten, tantalum, titanium, nickel, copper, iron, or an alloy of any combination thereof is preferred when the stretch resistant wire 12 is made of a metal material. For the same reason as in the case of the metal, polyethylene, polypropylene, nylon, polyester, polydioxanone, polytetrafluoroethylene, polyglycolic acid, polylactic acid, silk, or a composite material of any combination thereof is preferred when the stretch resistant wire 12 is made of a resin material.

The stretch resistant wire 12 may also carry at least one biochemical active material. The use of this feature makes it possible to further increase the amount of the biochemical active material in the vascular embolization device 1.

The biochemical active material may be the same as that used in the resin wire 20. The biochemical active material carried by the stretch resistant wire 12 may be the same as or different from that in the resin wire 20.

Examples of methods for allowing the stretch resistant wire 12 to carry the biochemical active material include, but are not limited to, immersion of the stretch resistant wire 12 in a biochemical active material solution and methods including applying or spraying the solution onto the stretch resistant wire 12 and removing the solvent by drying.

### (Tip)

The stretch resistant wire 12 should be fixed at the front end of the vascular embolization device 1 of the present invention. For this purpose, a tip 11 is preferably provided at the front end of the coil 10.

When the coil 10 is placed at the target site in the blood vessel, the coil 10 should be prevented from stretching. For this purpose, the stretch resistant wire 12 inserted in the inside of the coil 10 is preferably fixed at at least two sites including the rear end of the coil 10 and the tip 11 fixed at the front end of the coil 10.

As shown in FIG. 1, the tip 11 has a ring part 13 that is provided on the inside side of the coil 10 to fix the stretch resistant wire 12.

In addition, the outer surface of the tip 11 is preferably smooth sphere- or hemisphere-shaped in order to prevent any damage to the target site in the blood vessel.

The tip 11 may be formed by melting and shaping a front end portion of the wire of the coil 10 into a desired shape. Alternatively, a tip-forming member separate from the coil 10 may be fixed to the coil 10 with an adhesive or by heat welding to form the tip 11.

The ring part 13 may also be used to fix the resin wire 20 at the front end of the vascular embolization device 1. For example, in the vascular embolization device 1 shown in FIG. 3, one resin wire 20 is inserted into and folded back at the ring part 13 so that the front end of the resin wire 20 is fixed to the tip 11.

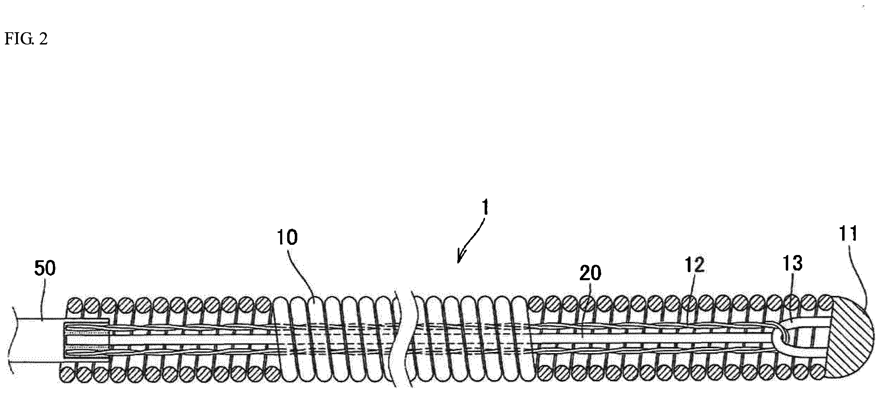

Alternatively, the resin wire 20 does not have to be fixed in the vascular embolization device 1 of the present invention. For example, in the vascular embolization device 1 shown in FIG. 1 or 2, the front end of the resin wire 20 is in contact with but not fixed to the tip 11.

FIGS. 1 to 3 show the vascular embolization device 1 in a linearly extended form. For example, when moved in a catheter, the vascular embolization device 1 takes this form. When not restrained by a catheter tube wall or other structure, the vascular embolization device 1 preferably takes the form of a secondary coil, in which the coil 10 is further wound as shown in FIG. 5.

In this regard, the diameter of the secondary coil, which is appropriately selected according to the inner diameter of the target site (e.g., aneurysm), is preferably from 1 mm to 40 mm, more preferably from 1.5 mm to 20 mm.

### (Pushing means)

In the vascular embolization device 1 of the present invention, the rear end of the coil 10 is attached, as shown in FIG. 6, to pushing means 30 with a connection part 50 interposed therebetween.

For example, as shown in FIGS. 1 to 3, the coil 10 is fixed to the connection part 50. In the vascular embolization device 1 shown in each of FIGS. 1 to 3, the coil 10 is fixed to the surface of a wire constituting the connection part 50.

The resin wire 20 may be fixed or not fixed to the connection part 50. For example, in the vascular embolization device 1 shown in each of FIGS. 2 and 3, the resin wire 20 is fixed to the surface of a wire constituting the connection part 50. On the other hand, in the vascular embolization device 1 shown in FIG. 1, the resin wire 20 is not fixed to the connection part 50.

To perform the stretch resistant function, the stretch resistant wire 12 is fixed to the connection part 50.

In this regard, the resin wire 20 and the stretch resistant wire 12 may be fixed to any position of the connection part 50, such as the surface or interior, and thus the position is not particularly limited.

Any known method such as adhesion or welding may be used to fix the connection part 50 to the coil 10, the resin wire 20, or the stretch resistant wire 12, and thus no limitation is imparted on the method.

In addition, the connection part 50 is so designed as to detachably connect the vascular embolization device 1.

Various methods may be used to detach the vascular embolization device 1. Typical detaching methods include, for example, detachment by thermally dissolving a resin wire of the connection part 50, detachment by electrolysis of a metal wire of the connection part 50, detachment by water pressure pushing, and detachment by mechanical unlocking. Among them, the method of detaching the coil by dissolving the wire of the connection part 50 is preferably used. Examples of the resin for forming the resin wire for coil separation include hydrophilic resins of synthetic polymers, including polyvinyl alcohol-based polymers such as polyvinyl alcohol (PVA), cross-linked PVA polymers, elastomers produced by freezing and thawing water absorption PVA gel, and ethylene-vinyl alcohol copolymers.

Among them, polyvinyl alcohol-based polymers are preferred because they can swell upon being in contact with water while maintaining a constant strength and can dissolve upon being heated at such a level as not to damage the living body.

FIG. 6 shows that pushing means 30 as an introducer is connected to the vascular embolization device 1 of the present invention (the vascular embolization device 1 with the features shown in FIG. 1). The pushing means 30 shown in FIG. 6 includes a wire portion 31 and a radiopaque distal portion 32 extending therefrom, in which the wire portion 31 includes a core wire and a resin coating layer formed on the outer surface of the core wire. The radiopaque distal portion 32 is connected and fixed to the rear end 50B of the resin wire constituting the connection part 50 fixed to the rear end 10B of the coil 10, so that the pushing means 30 is connected to the vascular embolization device 1. In this case, the rear end 10B of the coil 10 and the front end 50A of the resin wire constituting the connection part 50 for coil separation may be fixed to each other by any means, such as bonding with an adhesive, welding, connection by physical force, or other fixing means, and the radiopaque distal portion 32 of the pushing means 30 and the rear end 50B of the resin wire constituting the connection part 50 may also be fixed to each other by any means, such as bonding with an adhesive, welding, connection by physical force, or other fixing means.

The pushing means 30 preferably has an outer diameter of 0.1 mm to 2.0 mm. The pushing means 30 preferably has a length of 0.1 m to 2.0 m.

The core wire constituting the pushing means 30 is preferably a wire made of an electrically-conductive material such as stainless steel.

The resin coating layer in the wire portion 31 of the pushing means 30 can be formed by, for example, applying a fluororesin or a hydrophilic resin to the outer surface of the core wire. The resin coating layer made of a fluororesin or a hydrophilic resin is advantageous in that it can reduce the surface friction coefficient.

At the outer end of the wire portion 31, the core wire is exposed to form a terminal portion 33, through which electric power can be supplied to the core wire via an appropriate conductive member such as an electric connector, plugs, or clips. The terminal portion 33 with a length of about 1 cm to about 3 cm is long enough.

The radiopaque distal portion 32 of the pushing means 30 has a secondary form in which a winding wire is further wound into a coil on the outer surface of the core wire. A wire made of a metal such as platinum, silver, gold, tungsten, or stainless steel may be used for the winding wire that forms the radiopaque distal portion 32.

The vascular embolization device 1 of the present invention to which the pushing means 30 is connected as shown in FIG. 6 is introduced through any appropriate catheter to the target site in the living body.

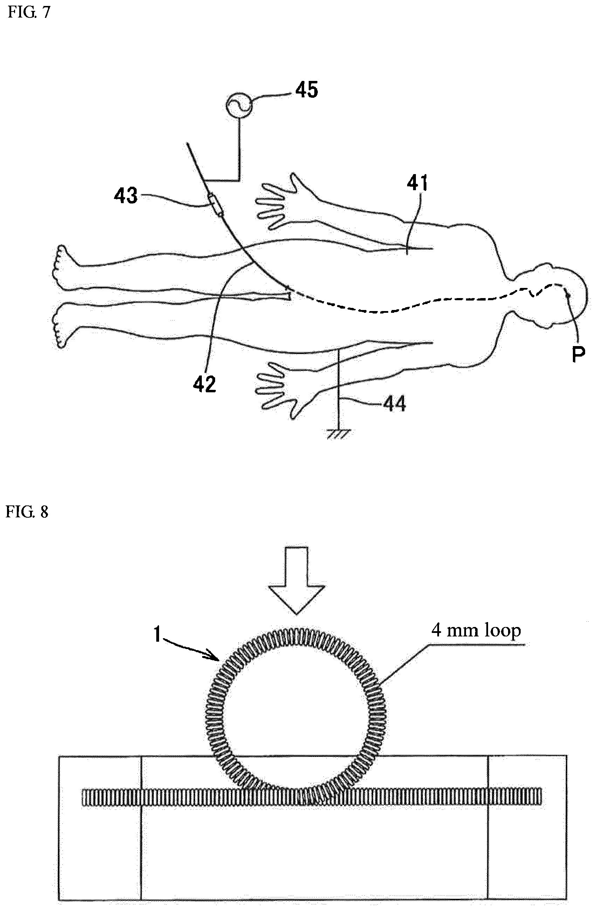

Specifically, as shown in FIG. 7, a catheter 42 is first inserted in such a manner that its front end opening reaches a target site P in the living body 41, and the vascular embolization device 1 as a front part is then inserted from a hand operation unit 43 into the catheter 42. Thus, the vascular embolization device 1 is moved in a linearly extended form through the catheter 42 while being pushed by the pushing means 30 and pushed out to the target site P from the front end opening of the catheter 42. When the connection part 50 reaches the front end opening of the catheter 42, an earth electrode 44 is attached to an appropriate skin surface of the living body 41, and, for example, a monopolar high-frequency current is supplied to the pushing means 30 from a high-frequency power supply 45 connected to the terminal portion 33 of the pushing means 30.

As a result, the high-frequency current generates heat to melt and cut off the rear end 50B of the resin wire constituting the connection part 50 between the vascular embolization device 1 and the pushing means 30, so that the vascular embolization device 1 is separated from the pushing means 30 and successfully placed at the target site P.

Therefore, when a resin with a melting point of 100°C or less is selected as a component of the resin wire constituting the connection part 50, the rear end 50B of the resin wire can be cut off in a short time by heating during high-frequency current supply.

Specifically, the resin wire constituting the connection part 50 may be made of a hydrophilic resin including a polyvinyl alcohol based polymer. In this case, the rear end 50B of the resin wire can be melted and cut off in a very short time of at most 3 seconds by supplying a high-frequency current.

This makes it possible to significantly reduce the burden on not only the operator but also the living body undergoing the operation and also makes it possible to significantly reduce the possibility of occurrence of unexpected events on the living body during the placement operation.

The vascular embolization device having the features described above can provide a good therapeutic effect by having good coil delivery performance, a good embolization function, and a drug delivery function.

Hereinafter, a method for producing the vascular embolization device of the present invention will be described, which, however, is not intended to limit the present invention.

A method for producing the vascular embolization device of the present invention includes the following three steps (a), (b), and (c).
(a) The step of coating the surface of a resin wire with a solution containing a biochemical active material and a resin composition
(b) The step of drying the solution to form a biochemical active material-containing layer on the surface of the resin wire as a core
(c) The step of inserting the resin wire with the biochemical active material-containing layer formed thereon into the inside of a coil to form a vascular embolization device

The details of the steps (a), (b), and (c) may be appropriately controlled according to the features of the vascular embolization device.

For example, the vascular embolization device 1 shown in FIGS. 1 and 6 (the vascular embolization device 1 with the resin wire 20 neither fixed to the tip 11 nor to the connection part 50) can be produced by the following steps.
(a1) A spinning step including performing melt spinning to form a resin wire for forming a core 21
(a2) A coating step including coating the surface of the resin wire (core 21) with a solution containing a biochemical active material and a resin composition
(b) A drying step including drying the solution to form a biochemical active material-containing layer (outer layer 22)
(c) An assembling step including inserting, into the inside of a coil 10, a stretch resistant wire 12 and the resin wire 20 with the biochemical active material-containing layer 22 formed thereon
(d) A tip forming step including forming a tip 11 at the front end of the coil 10
(e) A bonding step including bonding a resin wire (connection part 50) for coil separation to the base end of the coil 10 and to the front end of the wire of pushing means 30
(f) A trimming step including removing, by cutting, an excess part of the stretch resistant wire 12 protruding from the base end of the coil 10

Hereinafter, each step will be described in detail.

### (a1) Spinning step

A resin for forming a core 21 is melted by heating with a heater and spun into a fiber with a thickness of about 30 to about 150 µm. The resulting resin wire is taken up on a bobbin.

### (a2) Coating step

Using an X stage and a dispenser, a die, a spray, or other means, the resin wire placed linearly is subjected to coating.

### (b) Drying step

In a vacuum heating oven, the coating is dried under reduced pressure at 40 to 80°C for 1 to 24 hours.

### (c) Assembling step

Under microscopic observation, the stretch resistant wire 12 and the resin wire 20 with the biochemical active material-containing layer formed thereon are inserted into the inside of the coil 10.

### (d) Tip forming step

The part of the resin wire 20 protruding from the front end of the coil 10 is removed by cutting. Subsequently, using a YAG laser irradiation device, a tip 11 is formed by irradiating the coil 10 with laser light focused on the front end of the coil 10. In addition, a ring part 13 is formed on the side close to the inside of the coil 10, and the stretch resistant wire 12 is inserted into the ring part 13 and then folded back at the ring part 13.

### (e) Bonding step

Under microscopic observation, the part of the resin wire 20 protruding from the base end of the coil 10 is removed by cutting so that the resin wire 20 is completely inserted in the coil 10. Using a dispenser, a resin wire 50 for coil separation is bonded to the base end of the coil 10 with an instantaneous adhesive. At the same time, the stretch resistant wire 12 is also bonded to the surface of the resin wire 50 for coil separation. After the adhesive is cured, the resin wire 50 for coil separation, which extends from the coil 10, is similarly bonded to the front end of the wire of the pushing means 30 with an instantaneous adhesive.

### (f) Trimming step

Under microscopic observation, an excess part of the stretch resistant wire 12 protruding from the base end of the coil 10 is removed by cutting.

When the vascular embolization device 1 shown in FIG. 2 is produced, the base end-side part of the resin wire 20 may be bonded to the resin wire 50 for coil separation in the step (e) of the production method described above.

When the vascular embolization device 1 shown in FIG. 3 is produced, the resin wire 20 and the stretch resistant wire 12 may be inserted into and folded back at the ring part 13 of the tip 11 and both ends of the resin wire 20 may be placed at the base end of the coil 10 in the step (d) of the production method described above.

Both ends of the resin wire 20 may be bonded to, attached to, brought into contact with, or not in contact with the resin wire 50 for coil separation, which is disposed at the base end-side part of the coil 10.

Both ends of the stretch resistant wire 12 may also be fixed to the base end of the coil 10 by bonding, adhesion, or other methods.

The stranding machine, X stage, dispenser, vacuum heating oven, microscope, YAG laser irradiation device, instantaneous adhesive, and other means for use in the steps described above may be of any type useful for medical device production.

### EXAMPLES

Hereinafter, the present invention will be more specifically described with reference to examples. It will be understood that the examples below are not intended to limit the present invention.

### <Example 1>

Pellets of an ethylene-vinyl acetate copolymer with a vinyl acetate content of 25% (EVA25) were subjected to melt spinning to form a core wire with a thickness of about 0.10 mm.

A solution was prepared by dissolving 0.40 g of atorvastatin (AV) and 1.20 g of an ethylene-vinyl acetate copolymer with a vinyl acetate content of 40% (EVA40) in 24 mL of tetrahydrofuran. The surface of the wire was coated with the resulting solution at room temperature. The tetrahydrofuran was then evaporated to dryness by heating under reduced pressure, so that a drug-carrying resin wire was obtained.

In this process, the core wire was successfully coated with the solution without being broken because the EVA25 of the core wire with a vinyl acetate content of 25% was insoluble in tetrahydrofuran at room temperature, whereas the EVA40 with a vinyl acetate content of 40% used for the coating of the resin wire was soluble in tetrahydrofuran.

The cross-section of the prepared drug-carrying resin wire was observed with an electron microscope. As a result, it was observed that a drug-containing layer (outer layer) with a thickness of about 0.01 mm was formed on the surface of the base wire (core).

The drug-carrying resin wire with a thickness of 0.12 mm prepared in the same manner as in Example 1 and a stretch resistant wire (a stranded wire composed of two platinum-tungsten (Pt-W) alloy wires each with a diameter of 0.01 mm and a break strength of 0.4 N) were inserted in the inside of a metal coil with an element wire diameter of 0.045 mm, a primary coil diameter of 0.30 mm, and a secondary coil diameter of 4 mm.

Subsequently, the product was subjected to the steps (d), (e), and (f) described above, so that a vascular embolization device with the structure shown in FIGS. 1 and 6 was obtained.

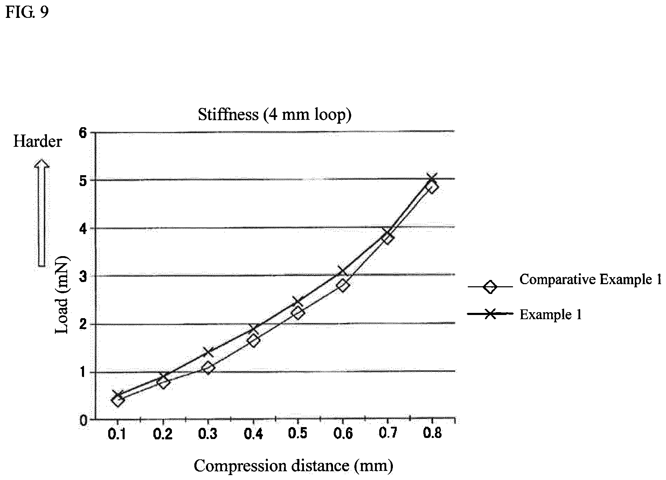

The resulting vascular embolization device 1 was evaluated for coil flexibility. In the evaluation, the vascular embolization device 1 was fixed in the form of a loop with a diameter of 4 mm, which was equal to the secondary diameter of the coil 10 to be tested, and the load required to compress the coil loop by a predetermined distance from the top was measured (FIG. 8). FIG. 9 shows the results.

### <Comparative Example 1>

A conventional vascular embolization device (bare coil) with no resin wire being inserted therein was prepared and then evaluated for coil flexibility as in Example 1. FIG. 9 shows the results.

The coil flexibility is compared at the same compression distance. A higher compression load means a higher coil stiffness and therefore a lower flexibility.

The measurement results of Example 1 and Comparative Example 1 show that the vascular embolization device 1 of Example 1 according to the present invention has a similar degree of flexibility to that of the vascular embolization device (bare coil) of Comparative Example 1 without any resin wire 20 being inserted therein, and thus can be safely placed at a desired target site in the blood vessel. The vascular embolization device of Example 1 also has a high embolization effect because it contains atorvastatin in the resin wire 20 disposed inside the coil 10 so that atorvastatin can be slowly released from the resin wire when it is placed in blood vessels.

### REFERENCE SIGNS LIST

- 10: Coil
- 11: Tip
- 12: Stretch resistant wire
- 13: Ring part
- 20: Resin wire
- 21: Core
- 22: Outer layer
- 30: Pushing means
- 31: Wire portion
- 32: Radiopaque distal portion
- 33: Terminal portion
- 41: Living body
- 42: Catheter
- 43: Hand operation unit
- 44: Earth electrode
- 45: High-frequency power supply
- 50: Connection part

## Claims

1. A vascular embolization device (1) comprising:
a coil (10); and
a resin wire (20) that contains a biochemical active material and is inserted in an inside of the coil (10), wherein
the biochemical active material is a blood coagulation accelerator, an anticancer drug, or any other drug,
the resin wire (20) is a multilayer strand including a core (21) and at least one outer layer (22), and
the core (21) and the at least one outer layer (22) individually contain resin compositions with solubilities different from each other in a same organic solvent,
**characterized in that**
the resin composition in at least one of the core (21) and the at least one outer layer (22) | includes an ethylene-vinyl acetate copolymer, a styrene-isobutylene-styrene block copolymer, a styrene-ethylene-propylene-styrene block copolymer, a styrene-ethylene-butadiene-styrene block copolymer, or a styrene-ethylene-ethylene-propylene-styrene block copolymer.

2. The vascular embolization device (1) according to claim 1, wherein the resin composition in the at least one outer layer (22) has a solubility higher than a solubility of the resin composition in the core (21).

3. The vascular embolization device (1) according to claim 1 or 2, wherein the resin composition in either the core (21) or the at least one outer layer (22) is composed of an ethylene-vinyl acetate copolymer.

4. The vascular embolization device (1) according to any one of claims 1 to 3, wherein the resin compositions in the outer layer (22) and the core (21) individually contain ethylene-vinyl acetate copolymers with compositions different from each other.

5. The vascular embolization device (1) according to claim 4, wherein the ethylene-vinyl acetate copolymer in the core (21) has a vinyl acetate unit content of 10 to 30% by weight, and the ethylene-vinyl acetate copolymer in the at least one outer layer (22) has a vinyl acetate unit content of 30 to 50% by weight.

6. The vascular embolization device (1) according to any one of claims 1 to 5, wherein the resin wire (20) is disposed in the inside of the coil (10) when the coil (10) is in a primary form.

7. The vascular embolization device (1) according to any one of claims 1 to 6, further comprising a stretch resistant wire (12) disposed in the inside of the coil (10) when the coil (10) is in a primary form.

8. The vascular embolization device (1) according to claim 7, wherein the stretch resistant wire (12) has a break strength of at least 0.05 N per wire.

9. The vascular embolization device (1) according to claim 7 or 8, wherein the stretch resistant wire (12) is corrugated or spirally shaped.

10. The vascular embolization device (1) according to any one of claims 7 to 9, wherein the stretch resistant wire (12) has a natural length at least 5% longer than a natural length of the coil (10).

11. The vascular embolization device (1) according to any one of claims 7 to 10, wherein the stretch resistant wire (12) is made of a metal.

12. The vascular embolization device (1) according to any one of claims 7 to 10, wherein the stretch resistant wire (12) is made of a resin.

13. The vascular embolization device (1) according to claim 11, wherein the stretch resistant wire (12) is made of gold, platinum, iridium, tungsten, tantalum, titanium, nickel, copper, iron, or an alloy of any combination thereof.

14. The vascular embolization device (1) according to claim 12, wherein the stretch resistant wire (12) is made of polyethylene, polypropylene, nylon, polyester, polydioxanone, polytetrafluoroethylene, polyglycolic acid, polylactic acid, silk, or a composite material of any combination thereof.

15. The vascular embolization device (1) according to any one of claims 1 to 14, wherein the biochemical active material is contained in the at least one outer layer (22) of the resin wire (20).

16. The vascular embolization device (1) according to any one of claims 1 to 15, wherein the biochemical active material is contained in an outermost layer of the resin wire (20).

17. The vascular embolization device (1) according to any one of claims 1 to 16, wherein the biochemical active material is contained in the core (21) of the resin wire (20).

18. The vascular embolization device (1) according to any one of claims 1 to 17, wherein the biochemical active material is a statin or includes a statin.

19. The vascular embolization device (1) according to claim 18, wherein the statin is simvastatin, pravastatin, atorvastatin, pitavastatin, or any combination thereof.

20. The vascular embolization device (1) according to any one of claims 1 to 19, wherein the resin wire (20) has a thickness of 0.01 to 0.20 mm.

21. The vascular embolization device (1) according to any one of claims 1 to 20, wherein the vascular embolization device (1) takes the form of a secondary coil when not restrained by a catheter tube wall or other structure.

22. A method for producing the vascular embolization device (1) according to any one of claims 1 to 21, the method comprising the three steps of:
(a) coating a surface of the core (21) with a solution containing the biochemical active material, the resin composition, and the organic solvent;
(b) drying the solution to form the resin wire (20) having thereon a layer (22) containing the biochemical active material; and
(c) inserting the resin wire (20) having thereon the layer (22) containing the biochemical active material, in the inside of the coil (10) to form the vascular embolization device (1).

## Patentansprüche

1. Gefäßembolisationsvorrichtung (1) mit:
einer Spirale (10); und
einem Harzdraht (20), der ein biochemisch aktives Material enthält und in ein Inneres der Spirale (10) eingeführt ist, wobei
das biochemisch aktive Material ein Blutgerinnungsbeschleuniger, ein Krebsmedikament oder ein anderes Medikament ist,
der Harzdraht (30) ein mehrschichtiger Strang ist, der einen Kern (21) und mindestens eine Außenschicht (22) aufweist, und
der Kern (21) und die mindestens eine Außenschicht (22) einzeln Harzzusammensetzungen mit voneinander verschiedenen Löslichkeiten in einem gleichen organischen Lösungsmittel enthalten,
**dadurch gekennzeichnet, dass**
die Harzzusammensetzung in mindestens einem von dem Kern (21) und der mindestens einen Außenschicht (22) ein Ethylen-Vinylacetat-Copolymer, ein Styrol-Isobutylen-Styrol-Blockcopolymer, ein Styrol-Ethylen-Propylen-Styrol-Blockcopolymer, ein Styrol-Ethylen-Butadien-Styrol-Blockcopolymer oder ein Styrol-Ethylen-Ethylen-Propylen-Styrol-Blockcopolymer umfasst.

2. Gefäßembolisationsvorrichtung (1) nach Anspruch 1, wobei die Harzzusammensetzung in der mindestens einen Außenschicht (22) eine Löslichkeit hat, die höher als eine Löslichkeit der Harzzusammensetzung in dem Kern (21) ist.

3. Gefäßembolisationsvorrichtung (1) nach Anspruch 1 oder 2, wobei die Harzzusammensetzung in entweder dem Kern (21) oder der mindestens einen Außenschicht (22) aus einem Ethylen-Vinylacetat-Copolymer besteht.

4. Gefäßembolisationsvorrichtung (1) nach einem der Ansprüche 1 bis 3, wobei die Harzzusammensetzungen in der Außenschicht (22) und dem Kern (21) einzeln Ethylen-Vinylacetat-Copolymere mit voneinander verschiedenen Zusammensetzungen enthalten.

5. Gefäßembolisationsvorrichtung (1) nach Anspruch 4, wobei das Ethylen-Vinylacetat-Copolymer in dem Kern (21) einen Vinylacetateinheitengehalt von 10 bis 30 Gewicht-% hat und das Ethylen-Vinylacetat-Copolymer in der mindestens einen Außenschicht (22) einen Vinylacetateinheitengehalt von 30 bis 50 Gewicht-% hat.

6. Gefäßembolisationsvorrichtung (1) nach einem der Ansprüche 1 bis 5, wobei der Harzdraht (20) in dem Inneren der Spirale (10) angeordnet ist, wenn die Spirale (10) in einer Primärform ist.

7. Gefäßembolisationsvorrichtung (1) gemäß einem der Ansprüche 1 bis 6, die außerdem einen dehnungsbeständigen Draht (12) umfasst, der in dem Inneren der Spirale (10) angeordnet ist, wenn die Spirale (10) in einer Primärform ist.

8. Gefäßembolisationsvorrichtung (1) nach Anspruch 7, wobei der dehnungsbeständige Draht (12) eine Bruchfestigkeit von mindestens 0,05 N pro Draht hat.

9. Gefäßembolisationsvorrichtung (1) nach Anspruch 7 oder 8, wobei der dehnungsbeständige Draht (12) gewellt oder spiralförmig ist.

10. Gefäßembolisationsvorrichtung (1) nach einem der Ansprüche 7 bis 9, wobei der dehnungsbeständige Draht (12) eine natürliche Länge hat, die mindestens 5% länger als eine natürliche Länge der Spirale (10) ist.

11. Gefäßembolisationsvorrichtung (1) nach einem der Ansprüche 7 bis 10, wobei der dehnungsbeständige Draht (12) aus einem Metall besteht.

12. Gefäßembolisationsvorrichtung (1) nach einem der Ansprüche 7 bis 10, wobei der dehnungsbeständige Draht (12) aus einem Harz besteht.

13. Gefäßembolisationsvorrichtung (1) nach Anspruch 11, wobei der dehnungsbeständige Draht (12) aus Gold, Platin, Iridium, Wolfram, Tantal, Titan, Nickel, Kupfer, Eisen oder einer Legierung einer beliebigen Kombination davon besteht.

14. Gefäßembolisationsvorrichtung (1) nach Anspruch 12, wobei der dehnungsbeständige Draht (12) aus Polyethylen, Polypropylen, Nylon, Polyester, Polydioxanon, Polytetrafluorethylen, Polyglykolsäure, Polymilchsäure, Seide oder einem Verbundmaterial einer beliebigen Kombination davon besteht.

15. Gefäßembolisationsvorrichtung (1) nach einem der Ansprüche 1 bis 14, wobei das biochemisch aktive Material in der mindestens einen Außenschicht (22) des Harzdrahts (20) enthalten ist.

16. Gefäßembolisationsvorrichtung (1) nach einem der Ansprüche 1 bis 15, wobei das biochemisch aktive Material in einer äußersten Schicht des Harzdrahts (20) enthalten ist.

17. Gefäßembolisationsvorrichtung (1) nach einem der Ansprüche 1 bis 16, wobei das biochemisch aktive Material in dem Kern (21) des Harzdrahts (20) enthalten ist.

18. Gefäßembolisationsvorrichtung (1) nach einem der Ansprüche 1 bis 17, wobei das biochemisch aktive Material ein Statin ist oder ein Statin enthält.

19. Gefäßembolisationsvorrichtung (1) nach Anspruch 18, wobei das Statin Simvastatin, Pravastatin, Atorvastatin, Pitavastatin oder eine beliebige Kombination davon ist.

20. Gefäßembolisationsvorrichtung (1) nach einem der Ansprüche 1 bis 19, wobei der Harzdraht (20) eine Dicke von 0,01 bis 0,20 mm hat.

21. Gefäßembolisationsvorrichtung (1) nach einem der Ansprüche 1 bis 20, wobei die Gefäßembolisationsvorrichtung (1) die Form einer Sekundärspirale einnimmt, wenn sie nicht durch eine Katheterschlauchwand oder einen anderen Aufbau eingeschränkt wird.

22. Verfahren zur Herstellung der Gefäßembolisationsvorrichtung (1) nach einem der Ansprüche 1 bis 21, wobei das Verfahren die folgenden drei Schritte umfasst:
(a) Beschichten einer Oberfläche des Kerns (21) mit einer Lösung, die das biochemisch aktive Material, die Harzzusammensetzung und das organische Lösungsmittel enthält;
(b) Trocknen der Lösung, um den Harzdraht (20) auszubilden, der darauf eine Schicht (22) hat, die das biochemisch aktive Material enthält; und
(c) Einführen des Harzdrahts (20), der darauf die Schicht (22) hat, die das biochemisch aktive Material enthält, in das Innere der Spirale (10), um die Gefäßembolisationsvorrichtung (1) auszubilden.

## Revendications

1. Dispositif d'embolisation vasculaire (1) comprenant :
une spirale (10) ; et
un fil en résine (20) qui contient un matériau actif biochimique et qui est inséré à l'intérieur de la spirale (10),
dans lequel
le matériau actif biochimique est un accélérateur de la coagulation du sang, un médicament anticancéreux, ou n'importe quel autre médicament,
le fil en résine (20) est un brin multicouche contenant un cœur (21) et au moins une couche extérieure (22), et
le cœur (21) et l'au moins une couche extérieure (22) contiennent individuellement des compositions de résine ayant des solubilités mutuellement différentes dans un même solvant organique,
**caractérisé en ce que** la composition de résine dans au moins l'un parmi le cœur (21) et l'au moins une couche extérieure (22) contient un copolymère d'éthylène-acétate de vinyle, un copolymère séquencé de styrène-isobutylène-styrène, un copolymère séquencé de styrène-éthylène-propylène-styrène, un copolymère séquencé de styrène-éthylène-butadiène-styrène, ou un copolymère séquencé de styrène-éthylène-éthylène-propylène-styrène.

2. Dispositif d'embolisation vasculaire (1) selon la revendication 1, dans lequel la composition de résine dans l'au moins une couche extérieure (22) a une solubilité supérieure à la solubilité de la composition de résine dans le cœur (21).

3. Dispositif d'embolisation vasculaire (1) selon la revendication 1 ou 2, dans lequel la composition de résine soit dans le cœur (21) soit dans l'au moins une couche extérieure (22) est composée d'un copolymère d'éthylène-acétate de vinyle.

4. Dispositif d'embolisation vasculaire (1) selon l'une quelconque des revendications 1 à 3, dans lequel les compositions de résine dans la couche extérieure (22) et le cœur (21) contiennent individuellement des copolymères d'éthylène-acétate de vinyle ayant des compositions mutuellement différentes.

5. Dispositif d'embolisation vasculaire (1) selon la revendication 4, dans lequel le copolymère d'éthylène-acétate de vinyle dans le cœur (21) a une teneur en motifs acétate de vinyle de 10 à 30 % en poids, et le copolymère d'éthylène-acétate de vinyle dans l'au moins une couche extérieure (22) a une teneur en motifs acétate de vinyle de 30 à 50 % en poids.

6. Dispositif d'embolisation vasculaire (1) selon l'une quelconque des revendications 1 à 5, dans lequel le fil en résine (20) est disposé à l'intérieur de la spirale (10) quand la spirale (10) est sous une forme primaire.

7. Dispositif d'embolisation vasculaire (1) selon l'une quelconque des revendications 1 à 6, comprenant en outre un fil résistant à l'étirement (12) disposé à l'intérieur de la spirale (10) quand la spirale (10) est sous une forme primaire.

8. Dispositif d'embolisation vasculaire (1) selon la revendication 7, dans lequel le fil résistant à l'étirement (12) a une résistance à la rupture d'au moins 0,05 N par fil.

9. Dispositif d'embolisation vasculaire (1) selon la revendication 7 ou 8, dans lequel le fil résistant à l'étirement (12) est ondulé ou en forme de spirale.

10. Dispositif d'embolisation vasculaire (1) selon l'une quelconque des revendications 7 à 9, dans lequel le fil résistant à l'étirement (12) a une longueur naturelle supérieure d'au moins 5 % à la longueur naturelle de la spirale (10).

11. Dispositif d'embolisation vasculaire (1) selon l'une quelconque des revendications 7 à 10, dans lequel le fil résistant à l'étirement (12) est fait en un métal.

12. Dispositif d'embolisation vasculaire (1) selon l'une quelconque des revendications 7 à 10, dans lequel le fil résistant à l'étirement (12) est fait en une résine.

13. Dispositif d'embolisation vasculaire (1) selon la revendication 11, dans lequel le fil résistant à l'usure (12) est fait en or, platine, iridium, tungstène, tantale, titane, nickel, cuivre, fer, ou un alliage de l'une quelconque de leurs combinaisons.

14. Dispositif d'embolisation vasculaire (1) selon la revendication 12, dans lequel le fil résistant à l'usure (12) est fait en polyéthylène, polypropylène, nylon, polyester, polydioxanone, polytétrafluoroéthylène, poly(acide glycolique), poly(acide lactique), soie, ou un matériau composite de l'une quelconque de leurs combinaisons.

15. Dispositif d'embolisation vasculaire (1) selon l'une quelconque des revendications 1 à 14, dans lequel le matériau actif biochimique est contenu dans l'au moins une couche extérieure (22) du fil en résine (20).

16. Dispositif d'embolisation vasculaire (1) selon l'une quelconque des revendications 1 à 15, dans lequel le matériau actif biochimique est contenu dans une couche la plus extérieure du fil en résine (20).

17. Dispositif d'embolisation vasculaire (1) selon l'une quelconque des revendications 1 à 16, dans lequel le matériau actif biochimique est contenu dans le cœur (21) du fil en résine (20) .

18. Dispositif d'embolisation vasculaire (1) selon l'une quelconque des revendications 1 à 17, dans lequel le matériau actif biochimique est une statine ou contient une statine.

19. Dispositif d'embolisation vasculaire (1) selon la revendication 18, dans lequel la statine est la simvastatine, la pravastatine, l'atorvastatine, la pitavastatine, ou l'une quelconque de leurs combinaisons.

20. Dispositif d'embolisation vasculaire (1) selon l'une quelconque des revendications 1 à 19, dans lequel le fil en résine (20) a une épaisseur de 0,01 à 0,20 mm.

21. Dispositif d'embolisation vasculaire (1) selon l'une quelconque des revendications 1 à 20, lequel dispositif d'embolisation vasculaire (1) prend la forme d'une spirale secondaire quand il n'est pas retenu par une paroi de tube de cathéter ou une autre structure.

22. Procédé pour produire le dispositif d'embolisation vasculaire (1) de l'une quelconque des revendications 1 à 21, le procédé comprenant les trois étapes suivantes :
(a) revêtement d'une surface du cœur (21) avec une solution contenant le matériau actif biochimique, la composition de résine, et le solvant organique ;
(b) séchage de la solution pour former le fil en résine (20) ayant sur celui-ci une couche (22) contenant le matériau actif biochimique ; et
(c) insertion du fil en résine (20), ayant sur celui-ci la couche (22) contenant le matériau actif biochimique, à l'intérieur de la spirale (10) pour former le dispositif d'embolisation vasculaire (1).
